# EUROPEAN PATENT APPLICATION

(11) **EP 4 566 546 A1**
(43) Date of publication of application: **11.06.2025**
(21) Application number: 24217609.7
(22) Date of filing: 04.12.2024
(51) Int. Cl.: A61B 8/06, A61B 8/00

(54) **ULTRASOUND DIAGNOSTIC DEVICE**

(30) Priority: 08.12.2023 JP 2023208084
(71) Applicant: Canon Medical Systems Corporation, Tochigi 324-0036 (JP)
(72) Inventor: IWASAKI, Ryosuke, Otawara-shi, 324-0036 (JP); KURITA, Koichiro, Otawara-shi, 324-0036 (JP)
(74) Representative: Marks & Clerk LLP

(57) **Abstract**

An ultrasound diagnostic device according to the present disclosure includes processing circuitry configured to acquire reflection ultrasound data from a mobile object; extract velocity information of the mobile object by applying a first filter to the reflection ultrasound data; determine a probability that the mobile object is a blood flow based on the reflection ultrasound data and output probability information; and generate a blood flow image based on the extracted velocity information of the mobile object and the outputted probability information.

## Description

### FIELD

The embodiments disclosed in the present specification and in the drawings relate to an ultrasound diagnostic device.

### BACKGROUND

In an ultrasound diagnostic device, information related to blood flow intensity and blood flow velocity is displayed on a display as a blood flow image that is represented by changes in color on reflection ultrasound data. When generating the blood flow image, there is a technique that uses color flow mapping (CFM) which assigns colors to the velocities of the blood flow. In such a technique, information such as a blood flow intensity, a blood flow velocity, and a blood flow direction is extracted by removing unwanted signals (clutter) contained in the reflection ultrasound data obtained from received reflected waves using a wall filter.

However, there may be cases where clutter cannot be completely removed by such a technique. Therefore, in order to prevent remaining clutter from impairing the visibility of the blood flow image, efforts such as assigning colors on a color map so that a portion with a low blood flow velocity is displayed in a dark color may be made. However, when using such a color map, blood flow with slower velocity is less likely to be displayed in the blood flow image.

When a blood flow with a low velocity is to be displayed, since clutter distributed in a low-velocity component in a velocity region and the blood flow overlap with each other, the clutter and the blood flow must be separated. To this end, recently, eigenvalue expansion-type wall filters have been used to separate a low-velocity blood flow from clutter. However, eigenvalue expansion-type wall filters have a problem in that a validity of a value related to a blood flow velocity estimated after filtering is not guaranteed due to filter characteristics being variable depending on input.

### SUMMARY OF INVENTION

An ultrasound diagnostic device may include processing circuitry configured to
acquire reflection ultrasound data from a mobile object;
extract velocity information of the mobile object by applying a first filter to the reflection ultrasound data;
determine a probability that the mobile object is a blood flow based on the reflection ultrasound data and output probability information; and
generate a blood flow image based on the extracted velocity information of the mobile object and the outputted probability information.

The processing circuitry may be further configured to
extract intensity information of the mobile object by applying a second filter to the reflection ultrasound data; and
determine a probability that the mobile object is the blood flow based on the intensity information.

The processing circuitry may be further configured to determine that the higher the intensity indicated by the intensity information is, the higher the probability of being the blood flow is.

The processing circuitry may be further configured to generate the blood flow image so that the higher the probability of being the blood flow is, the higher the brightness is, and the lower the probability of being the blood flow is, the lower the brightness is.

The processing circuitry may be further configured not to express a difference in velocity in the velocity information of the mobile object in terms of a difference in brightness.

The processing circuitry may be further configured to generate the blood flow image using a color map in which a first axis indicates the probability of being the blood flow and a second axis indicates the velocity of the mobile object.

The processing circuitry may be further configured to extract direction information in addition to the velocity information of the mobile object by applying the first filter to the reflection ultrasound data.

In the color map, a first color may be assigned to the mobile object moving in a direction approaching an ultrasound probe, a second color may be assigned to the mobile object moving in a direction receding from the ultrasound probe, a third color may be assigned to the mobile object of which a velocity of movement is zero, and a color scheme may be adopted such that the first color, the second color, and the third color gradually change based on a probability of being a blood flow and the velocity of the mobile object.

In the color map, the first color may be assigned to the mobile object of which a velocity of movement is zero, the second color may be respectively assigned to maximum values on the color map related to the velocity of the mobile object moving in a direction approaching an ultrasound probe and the velocity of the mobile object moving in a direction receding from the ultrasound probe, and a color scheme may be adopted such that a color changes to the second color via the third color as the velocity of the mobile object increases in the direction approaching the ultrasound probe and a color changes to the second color via a fourth color as the velocity of the mobile object increases in the direction receding from the ultrasound probe.

The first filter may be a fixed-length wall filter in a Doppler frequency space, and
the second filter may be an eigenvalue expansion-type wall filter.

### BRIEF DESCRIPTION OF THE DRAWINGS

FIG. 1 is a block diagram showing an example of an overall configuration of an ultrasound diagnostic device according to the present embodiment;
FIG. 2 is a block diagram showing an example of functions of a Doppler processing circuit included in the ultrasound diagnostic device according to the present embodiment;
FIG. 3 is a block diagram for describing processing contents of an image generation circuit in the ultrasound diagnostic device according to the present embodiment;
FIG. 4 is a diagram showing an example of a color map used when the image generation circuit in the ultrasound diagnostic device according to the present embodiment generates a blood flow image;
FIG. 5 is a diagram showing another example of a color map used when the image generation circuit in the ultrasound diagnostic device according to the present embodiment generates a blood flow image;
FIG. 6 is a diagram showing an example of a blood flow image displayed on a display of the ultrasound diagnostic device according to the present embodiment;
FIG. 7 is a diagram showing, as a comparative example, a blood flow image generated using a color map related to velocity information extracted by applying a wall filter for velocity/direction to reflection ultrasound data;
FIG. 8 is a diagram showing a flowchart for describing contents of blood flow image display processing executed by the ultrasound diagnostic device according to the present embodiment; and
FIG. 9 is a diagram showing a flowchart for describing contents of blood flow possibility determination processing executed by the ultrasound diagnostic device according to the present embodiment.

### DETAILED DESCRIPTION

Hereinafter, an embodiment of an ultrasound diagnostic device will be described with reference to the drawings. In the following description, constituent elements with substantially a same function and a same configuration will be denoted by a same reference sign and overlapping descriptions will only be given when necessary.

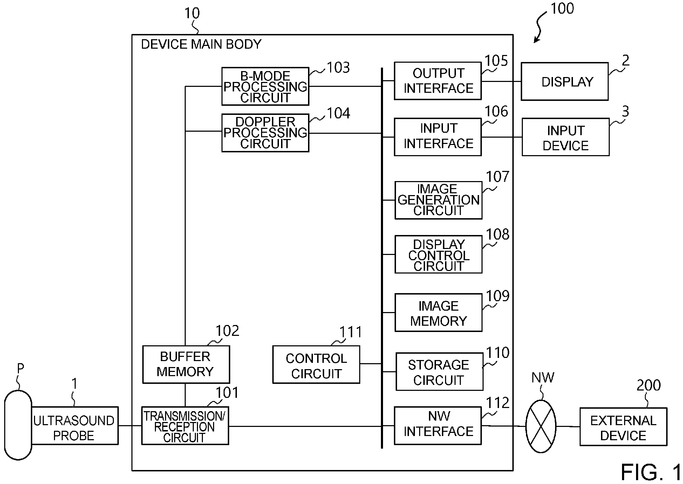

FIG. 1 is a block diagram showing an example of an ultrasound diagnostic device 100 according to the present embodiment. As shown in FIG. 1, the ultrasound diagnostic device 100 includes a device main body 10 and an ultrasound probe 1, a display 2, and an input device 3 connected to the device main body 10.

The device main body 10 is configured to include a transmission/reception circuit 101, a buffer memory 102, a B-mode processing circuit 103, a Doppler processing circuit 104, an output interface 105, an input interface 106, an image generation circuit 107, a display control circuit 108, an image memory 109, a storage circuit 110, a control circuit 111, and an NW (network) interface 112. In addition, the device main body 10 is connected to an external device 200 via a network NW.

For example, the ultrasound probe 1 includes a plurality of elements such as a piezoelectric transducer. The plurality of elements generate ultrasonic waves based on a drive signal supplied from the transmission/reception circuit 101 of the device main body 10. In addition, the ultrasound probe 1 receives reflected waves from a subject P and converts the reflected waves into an electric signal. Furthermore, for example, the ultrasound probe 1 includes a matching layer provided on the piezoelectric transducer and a backing material that prevents propagation of ultrasonic waves backward from the piezoelectric transducer. The ultrasound probe 1 is detachably connected to the device main body 10.

More specifically, when ultrasonic waves are transmitted from the ultrasound probe 1 to the subject P, the transmitted ultrasonic waves are reflected one after another at a discontinuity of acoustic impedance in a body tissue of the subject P and are received as a reflected wave signal by the plurality of elements in the ultrasound probe 1. An amplitude of the received reflected wave signal depends on a difference in acoustic impedance at the discontinuity where the ultrasonic waves are reflected. The reflected wave signal when a transmitted ultrasound pulse is reflected by a surface such as a moving blood stream or a heart wall undergoes a frequency shift that depends on a velocity component relative to an ultrasound transmission direction of the mobile object due to a Doppler effect. In addition, the ultrasound probe 1 outputs the reflected wave signal to the transmission/reception circuit 101 of the device main body 10.

In the present embodiment, the ultrasound probe 1 is, for example, a one-dimensional array probe that is an array of a plurality of ultrasound transducers in a predetermined direction. However, the ultrasound probe 1 is not limited to a one-dimensional array probe and may be a two-dimensional array probe (a probe in which a plurality of ultrasound transducers are arrayed in a two-dimensional matrix) or a mechanical 4D probe (a probe capable of executing an ultrasound scan while mechanically agitating a row of ultrasound transducers in a direction orthogonal to the array direction).

The input device 3 is realized by input means such as a mouse, a keyboard, buttons, panel switches, a touch command screen, foot switches, a trackball, a joystick, and the like. The input device 3 accepts various setting requests from an operator of the ultrasound diagnostic device 100 and forwards the various accepted setting requests to the device main body 10.

For example, the display 2 displays a GUI (Graphical User Interface) for the operator of the ultrasound diagnostic device 100 to input various setting requests using the input device 3 and displays ultrasound images and the like indicated by ultrasound image data generated in the device main body 10. The display 2 is realized by a liquid crystal monitor, a CRT (Cathode Ray Tube) monitor, or the like. The display 2 is an example of the displayer.

Under control of the control circuit 111, the transmission/reception circuit 101 causes the ultrasound probe 1 to transmit ultrasonic waves and receive ultrasonic waves (reflected waves of ultrasonic waves). In other words, the transmission/reception circuit 101 executes an ultrasound scan via the ultrasound probe 1.

More specifically, under control of the control circuit 111, the transmission/reception circuit 101 causes the ultrasound probe 1 to transmit ultrasonic waves. For example, the transmission/reception circuit 101 includes a trigger generation circuit, a delay circuit, a pulser circuit, and the like which are not illustrated. In the trigger generation circuit, trigger pulses for forming transmission ultrasonic waves at a predetermined rate frequency fr Hz are repeatedly generated. In addition, in the delay circuit, each trigger pulse is given a delay time necessary to focus the ultrasonic waves into a beam for each channel and to determine the directionality of the transmission. The pulser circuit applies a drive pulse to the ultrasound probe 1 at timings based on the trigger pulses.

In addition, the transmission/reception circuit 101 generates reflection ultrasound data that is ultrasound data based on the reflected wave signal received by the ultrasound probe 1. The transmission/reception circuit 101 stores the generated reflection ultrasound data in the buffer memory 102.

More specifically, after reaching the piezoelectric transducer inside the ultrasound probe 1, the reflected waves of ultrasonic waves transmitted by the ultrasound probe 1 are converted from mechanical vibrations into an electrical signal (reflected wave signal) by the piezoelectric transducer and inputted to the transmission/reception circuit 101. For example, the transmission/reception circuit 101 includes, for example, a pre-amplifier, an A/D (Analog-to-Digital) converter, and a quadrature detection circuit and performs various kinds of processing on the reflected wave signal received by the ultrasound probe 1 to generate reflection ultrasound data. In the present embodiment, the expression "acquiring reflection ultrasound data" includes obtaining reflection ultrasound data from a mobile object by transmission and reception of ultrasonic waves. The transmission/reception circuit 101 is an example of the acquirer according to the present embodiment.

The buffer memory 102 at least temporarily stores the reflection ultrasound data generated by the transmission/reception circuit 101. For example, the buffer memory 102 stores reflection ultrasound data acquired by pluralities of transmissions and receptions of ultrasonic waves per raster. The buffer memory 102 is realized by, for example, a semiconductor memory element such as a RAM (Random Access Memory) or a flash memory.

The B-mode processing circuit 103 performs processing such as logarithmic amplification, envelope detection, or logarithmic compression on the reflection ultrasound data read from the buffer memory 102 to generate data (B-mode data) in which a signal intensity is expressed in terms of a degree of brightness.

The Doppler processing circuit 104 performs a frequency analysis of the reflection ultrasound data stored in the buffer memory 102 to generate data (Doppler data) that is an extraction of motion information based on a Doppler effect of a mobile object present in an ROI (Region of Interest) set in a scan region. The mobile object is, for example, blood. For example, the Doppler processing circuit 104 is capable of executing a color Doppler method that is also known as a color flow mapping (CFM) method.

The output interface 105 outputs an electric signal from the control circuit 111 to the outside. For example, the output interface 105 is connected to the control circuit 111 via a bus and outputs an electric signal from the control circuit 111 to the display 2.

The input interface 106 accepts various instructions from the operator via the input device 3. For example, the input interface 106 is connected to the control circuit 111 via a bus, converts an operation instruction inputted by the operator into an electric signal, and outputs the electric signal to the control circuit 111. The input interface 106 is not limited to those connected to physical operation components such as a mouse and a keyboard. For example, a circuit that receives an electrical signal corresponding to an operation instruction inputted from an external input device provided separately from the ultrasound diagnostic device 100 and outputs the electrical signal to the control circuit 111 is also an example of an input interface.

The image generation circuit 107 generates ultrasound image data based on the data generated by the B-mode processing circuit 103 and the Doppler processing circuit 104. The image generation circuit 107 causes the generated ultrasound image data to be stored in the image memory 109.

More specifically, the image generation circuit 107 generates B-mode image data based on the B-mode data generated by the B-mode processing circuit 103. In addition, the image generation circuit 107 generates Doppler image data based on the Doppler data generated by the Doppler processing circuit 104. The Doppler image data is an example of data for displaying a blood flow image in the present embodiment. The image generation circuit 107 generates the Doppler image data based on intensity information and phase-change information included in the Doppler data generated by the Doppler processing circuit 104.

For example, the Doppler image data is velocity image data, dispersion image data, power image data, or image data that combines these pieces of image data. For example, from the Doppler data as blood flow information, the image generation circuit 107 generates data of a blood flow image in which the blood flow information is displayed in color as the Doppler image data. In this case, the image generation circuit 107 visualizes a blood flow as Doppler image data by determining a drawing position based on a signal intensity of the blood flow and determining a display color based on a velocity and a direction of the blood flow. The image generation circuit 107 is an example of the blood flow image generator according to the present embodiment.

The display control circuit 108 causes the display 2 to display an ultrasound image based on various kinds of ultrasound image data generated by the image generation circuit 107. In addition, the display control circuit 108 may cause the display 2 to display a GUI for the operator to input various setting requests using the input device 3.

The image memory 109 stores various kinds of image data generated by the control circuit 111. For example, the image memory 109 is realized by a semiconductor memory element such as a RAM or a flash memory, a hard disk, an optical disk, or the like.

For example, the storage circuit 110 is realized by a storage medium readable by a processor such as a magnetic or optical storage medium, a semiconductor memory element such as flash memory, a hard disk, or an optical disk. The storage circuit 110 stores programs, various kinds of data, and the like for realizing ultrasound transmission and reception. For example, the programs and various kinds of data may be stored in the storage circuit 110 in advance. In addition, for example, the programs and various kinds of data may be stored and distributed on a non-transitory storage medium, read from the non-transitory storage medium, and installed in the storage circuit 110. The storage circuit 110 may be considered an example of the storage unit according to the present embodiment.

The control circuit 111 exerts overall control over the operations of the entire ultrasound diagnostic device 100. For example, the control circuit 111 controls the ultrasound probe 1 via the transmission/reception circuit 101 to control ultrasound scanning and cause reflection ultrasound data to be acquired.

The NW interface 112 is connected to the external device 200 via, for example, the network NW and performs data communication with the external device 200.

For example, the external device 200 is a workstation that executes post-processing of various kinds of data generated by the ultrasound diagnostic device 100 and processing such as displaying ultrasound image data. For example, the external device 200 includes an NW interface that is capable of connecting to a processing circuit such as a processor, a storage device, a display, an input device, and the ultrasound diagnostic device 100 via the network NW. In addition, the external device 200 may be a tablet terminal or the like.

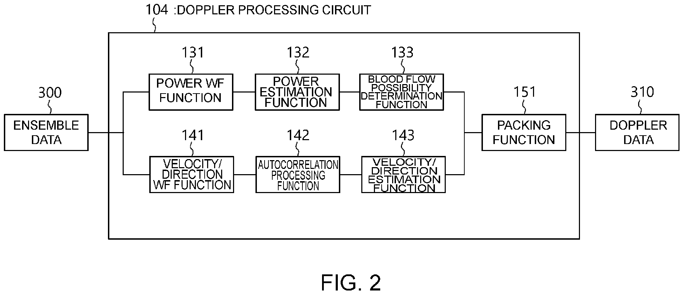

Next, the Doppler processing circuit 104 will be described in detail. FIG. 2 is a block diagram showing an example of functions of the Doppler processing circuit 104 according to the present embodiment. As shown in FIG. 2, the Doppler processing circuit 104 is configured so as to include a power WF (Wall Filter) function 131, a power estimation function 132, a blood flow possibility determination function 133, a velocity/direction WF function 141, an autocorrelation processing function 142, a velocity/direction estimation function 143, and a packing function 151.

The power WF function 131 and the velocity/direction WF function 141 respectively apply wall filters to ensemble data 300 that is reflection ultrasound data stored in the buffer memory 102.

More specifically, the power WF function 131 extracts intensity information related to a mobile object by applying a wall filter for power (intensity) information to the ensemble data 300. The power WF function 131 uses an eigenvalue expansion-type wall filter. The power WF function 131 is an example of the second filter processor according to the present embodiment. In addition, the eigenvalue expansion-type wall filter applied to the ensemble data 300 by the power WF function 131 is an example of the second filter according to the present embodiment.

For example, the eigenvalue expansion-type wall filter is an adaptive MTI (Moving Target Indicator) filter that uses an eigenvector to vary a coefficient according to an input signal. The eigenvector is calculated from a correlation matrix. Specifically, the power WF function 131 calculates a correlation matrix for a scan range from a data sequence of reflection ultrasound data at a same location having been collected over a plurality of frames. The power WF function 131 calculates a eigenvector from the correlation matrix and determines a coefficient to be used in the MTI filter based on the calculated eigenvector. The adaptive MTI filter using an eigenvector is an example of an eigenvalue expansion-type wall filter and other methods may be adopted.

The eigenvalue expansion-type wall filter detects a component with highest energy in an eigenvector dimension of the ensemble data 300 as a principal component and suppresses a Doppler shift that spatially approximates the principal component as a clutter component. Such an eigenvalue expansion-type wall filter is superior in terms of suppressing clutter and extracting a power component of a blood flow. On the other hand, since the characteristics of the filter vary according to the correlation matrix of the scan range, it cannot be said that information in any specific velocity range has been extracted when velocity information and direction information are estimated for a signal after passing through the filter.

The power estimation function 132 estimates intensity information of the mobile object from a component extracted from the ensemble data 300 by the power WF function 131. The power estimation function 132 is an example of the power estimator according to the present embodiment.

The blood flow possibility determination function 133 determines a probability that the mobile object is a blood flow based on the intensity information of the mobile object estimated by the power estimation function 132. A determination result thereof is outputted from the blood flow possibility determination function 133 as probability information. For example, in the present embodiment, the probability information is generated such that the higher the intensity indicated by the intensity information of the moving object is, the higher the probability information becomes. In other words, in the present embodiment, it is determined that the higher the intensity of the mobile object is, the higher the probability of the mobile object being a blood flow is. This is because, in general, when the mobile object is a tissue such as an organ or simply noise, the intensity of its reflection ultrasound data is conceivably weak. The blood flow possibility determination function 133 is an example of the determiner according to the present embodiment.

The velocity/direction WF function 141 extracts phase-change information by applying a wall filter for velocity/direction to the ensemble data 300. The velocity/direction WF function 141 uses a fixed-length wall filter in a Doppler frequency space. The phase-change information includes velocity information and direction information of the mobile object. The velocity/direction WF function 141 is an example of the first filter processor that extracts velocity information of the mobile object according to the present embodiment. In addition, the fixed-length wall filter used by the velocity/direction WF function 141 is an example of the first filter according to the present embodiment.

More specifically, in the present embodiment, the velocity/direction WF function 141 uses a polynomial approximation-type wall filter. In the polynomial approximation-type wall filter, a clutter component is removed by a polynomial fitting technique that performs fitting with a predetermined polynomial and specifies a component of a lower order as the clutter component. Note that a fixed-length wall filter other than a polynomial approximation-type wall filter may be adopted. For example, the velocity/direction WF function 141 may use an IIR (Infinite Impulse Response) filter.

In a fixed-length wall filter, a component with a low Doppler shift frequency is considered clutter and is suppressed. The Doppler shift frequency is lower for reflection ultrasound data derived from a stationary tissue or a slow-moving tissue. The fixed-length wall filter may have a lower ability to separate clutter and blood flow as compared to an eigenvalue expansion-type wall filter. However, since filter characteristics of the fixed-length wall filter are uniquely defined, changes in the velocity information and the direction information of a blood flow within a filter passage velocity range can be estimated with validity.

The autocorrelation processing function 142 performs autocorrelation processing with respect to data extracted from the ensemble data 300 by the velocity/direction WF function 141. The autocorrelation processing function 142 is an example of the autocorrelation processor.

The velocity/direction estimation function 143 estimates velocity information and direction information of the mobile object based on a result of the autocorrelation processing by the autocorrelation processing function 142. The velocity/direction estimation function 143 is an example of the velocity/direction estimator. The velocity/direction estimation function 143 may also estimate dispersion of the mobile object. When the velocity/direction estimation function 143 also estimates dispersion of the mobile object, the velocity/direction estimation function 143 also generates dispersion information.

The packing function 151 combines the probability information outputted from the blood flow possibility determination function 133 with the velocity information and the direction information of the mobile object estimated by the velocity/direction estimation function 143 to generate Doppler data 310 including probability information, velocity information, and direction information of the mobile object. A blood flow image is generated by the image generation circuit 107 from the Doppler data 310 generated by the packing function 151. When dispersion information is also generated by the velocity/direction estimation function 143, the packing function 151 generates the Doppler data 310 by also combining the dispersion information in addition to the probability information, the velocity information, and the direction information of the mobile object. The packing function 151 is an example of the packer according to the present embodiment.

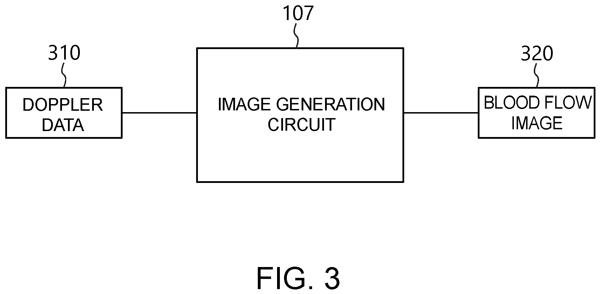

Next, a process by which the image generation circuit 107 generates a blood flow image will be described in detail. FIG. 3 is a block diagram for describing processing contents of the image generation circuit 107 in the device main body 10 of the ultrasound diagnostic device 100 according to the present embodiment. As shown in FIG. 3, the image generation circuit 107 according to the present embodiment acquires the Doppler data 310 outputted by the packing function 151 of the Doppler processing circuit 104. In the present embodiment, since the Doppler data 310 is also generated from reflection ultrasound data, the reflection ultrasound data may sometimes be synonymous with Doppler data 310.

More specifically, the image generation circuit 107 acquires the Doppler data 310 and acquires the probability information, the velocity information, and the direction information included in the Doppler data 310. A blood flow image 320 is generated based on the acquired probability information, velocity information, and direction information. However, in the present embodiment, the image generation circuit 107 generates the blood flow image 320 so that the higher the probability of being a blood flow in the probability information is, the higher the brightness is, and, conversely, the lower the probability is, the lower the brightness is. For example, the blood flow image 320 generated by the image generation circuit 107 is temporarily stored in the image memory 109 and displayed on the display 2.

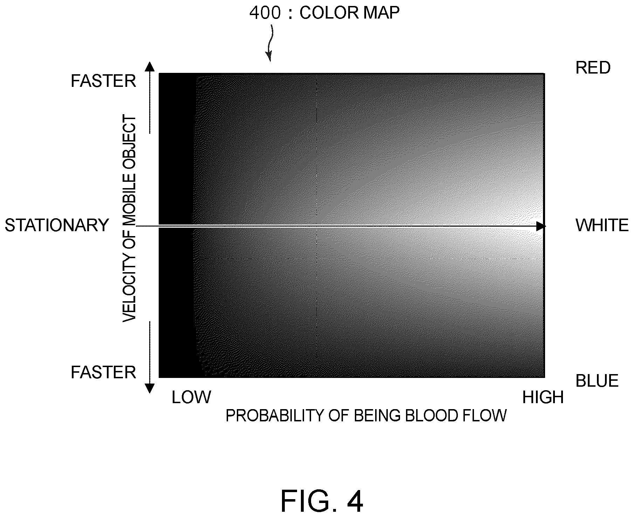

FIG. 4 is a diagram showing an example of a color map 400 used when the image generation circuit 107 generates the blood flow image 320 based on probability information, velocity information, and direction information. In the present embodiment, for example, the color map 400 may be held by the image generation circuit 107 itself. Alternatively, the color map 400 may be stored in the storage circuit 110 and the image generation circuit 107 may read the color map 400 from the storage circuit 110 and use the color map 400.

In the color map 400, for example, an axis of abscissa indicates a probability that the mobile object is a blood flow and an axis of ordinate indicates a velocity of the mobile object. The probability of being a blood flow in the color map 400 is determined based on the probability information included in the Doppler data 310. The velocity of the mobile object in the color map 400 is determined based on the velocity information and the direction information included in the Doppler data 310. While the axis of abscissa corresponds to the first axis and the axis of ordinate corresponds to the second axis in the present embodiment, the relationship between the axis of ordinate and the axis of abscissa is interchangeable.

In addition, in the example shown in FIG. 4, a color scheme is adopted such that chroma of red increases as the velocity of the mobile object moving in a direction approaching the ultrasound probe 1 increases and chroma of blue increases as the velocity of the mobile object moving in a direction receding from the ultrasound probe 1 increases. In other words, in the color map 400, a difference between high/low velocities of the mobile object is not expressed in terms of a difference in brightness. Furthermore, in the example shown in FIG. 4, a color scheme is adopted such that the higher the probability of being a blood flow is, the higher the brightness is. Therefore, in a blood flow image, the lower the probability of being a blood flow in a region, the closer the color displayed in the region to black.

Furthermore, in the example shown in FIG. 4, white is assigned to the mobile object of which the velocity is zero. Therefore, even when the velocity of the mobile object is zero or, in other words, even when the mobile object is stationary, the region is displayed by white if the probability of being a blood flow is high. Therefore, in a blood flow image, when the probability of being a blood flow is high, white or a color close to white is displayed even in a region where the velocity of the mobile object is low. Note that, in this case, white is an example of a bright color and, for example, yellow can be used in place of white in the color map 400.

In the color map 400 shown in FIG. 4, red that is a first color is assigned to the mobile object moving in a direction approaching the ultrasound probe 1, blue that is a second color is assigned to the mobile object moving in a direction receding from the ultrasound probe 1, and white that is a third color is assigned to the mobile object of which a velocity of movement is zero. In addition, a color scheme is adopted such that red being the first color, blue being the second color, and white being the third color gradually change based on a probability of being a blood flow and a velocity of the mobile object.

Due to the image generation circuit 107 generating a blood flow image based on the color map 400, even blood flow with low velocity is displayed while suppressing clutter and blood flow velocity is displayed with validity. Specifically, a clutter component is removed and suppressed by having the velocity/direction WF function 141 apply a fixed-length wall filter to the ensemble data 300 that is reflection ultrasound data. In addition, since the image generation circuit 107 generates a blood flow image based on the color map 400, white is arranged in the blood flow image even in a region where the velocity of blood flow is low such as a micro blood flow if the probability of being a blood flow is high. Therefore, even a fine blood flow or a blood flow with a low velocity can be displayed on a blood flow image. Furthermore, red or blue can be used in a region where the velocity of the mobile object is high and blood flow velocity can be displayed with validity.

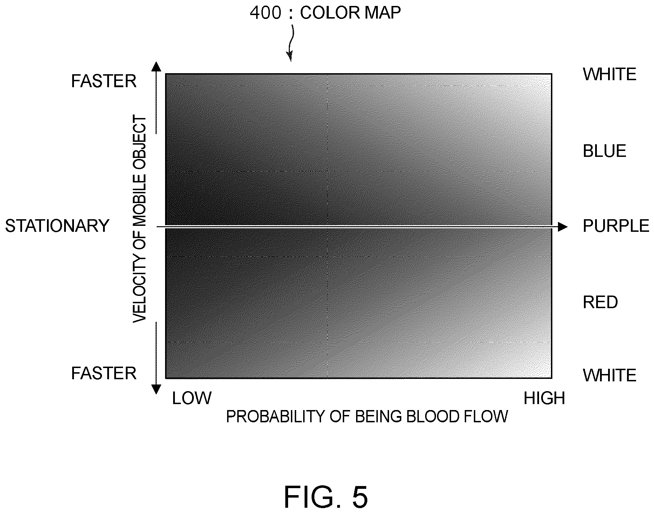

FIG. 5 is a diagram showing another example of the color map 400. The example shown in FIG. 5 also adopts a color scheme such that the higher the probability of being a blood flow is, the higher the brightness is. Therefore, in a blood flow image, the lower the probability of being a blood flow in a region, the more the region is displayed in black or a near-black color. On the other hand, in the example shown in FIG. 5, even when the velocity of the mobile object is zero or, in other words, even when the mobile object is stationary, purple is used if the probability of being a blood flow is high. Therefore, in a blood flow image, when the probability of being a blood flow is high, purple or a near-purple color is displayed even in a region where the velocity of the mobile object is low.

In addition, in the example shown in FIG. 5, a color scheme is adopted such that purple gradually becomes blue and then gradually becomes white as the velocity in a direction approaching the ultrasound probe 1 increases. Furthermore, a color scheme is adopted such that purple gradually becomes red and then gradually becomes white as the velocity in a direction receding from the ultrasound probe 1 increases.

In other words, in the color map 400 shown in FIG. 5, purple that is a first color is assigned to the mobile object of which a velocity of movement is zero and white that is a second color is respectively assigned to maximum values on the color map 400 related to velocities of the mobile object moving in a direction approaching the ultrasound probe 1 and moving in a direction receding from the ultrasound probe 1. In addition, a color scheme is adopted such that a color changes to white being the second color via blue that is a third color as the velocity of the mobile object increases in the direction approaching the ultrasound probe 1 and a color changes to white being the second color via red that is a fourth color as the velocity of the mobile object increases in the direction receding from the ultrasound probe 1.

Even when using the color map 400 with the color scheme shown in FIG. 5, the image generation circuit 107 can display blood flow velocity with validity and display a blood flow with low velocity while suppressing clutter. This is because the color map 400 according to the present embodiment adopts a color scheme such that the higher the probability of being a blood flow is, the higher the brightness is. Accordingly, display intensity of a blood flow image generated by the image generation circuit 107 can be adjusted to display blood flows in colors of appropriate velocity values while displaying blood flows with lower velocity. Therefore, any color combination can be used for the color scheme of the color map 400 as long as the color combination satisfies a condition that the higher the probability of being a blood flow is, the higher the brightness is.

The B-mode processing circuit 103, the Doppler processing circuit 104, the image generation circuit 107, the display control circuit 108, and the control circuit 111 are realized by processing circuitry. For example, computer-executable programs defining processing to be executed by the processing circuitry are stored in the storage circuit 110. The processing circuitry realize a function corresponding to each program by reading and executing the program from the storage circuit 110. In addition, while a single storage circuit 110 has been described with reference to FIG. 1 as storing the programs corresponding to the respective processing functions, a configuration may be adopted in which a plurality of storage circuits are arranged in a distributed manner and each circuit reads a corresponding program from an individual storage circuit.

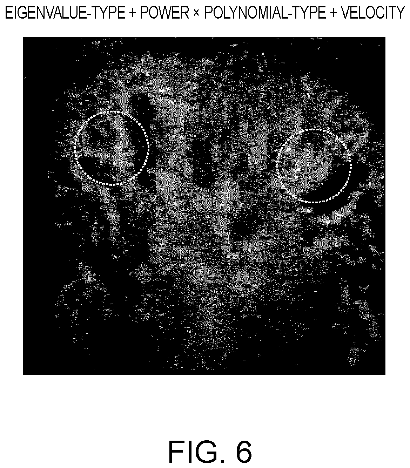

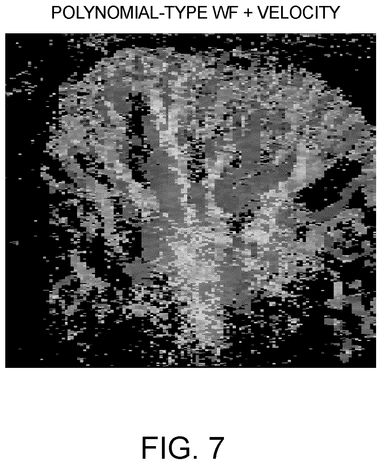

FIG. 6 is a diagram showing an example of the blood flow image 320 displayed on the display 2 of the ultrasound diagnostic device 100 according to the present embodiment, and FIG. 7 is a diagram showing, as a comparative example, a blood flow image generated in a conventional manner using a color scheme based on a color map related to velocity information acquired by applying a wall filter for velocity/direction to the ensemble data 300 that is reflection ultrasound data. While the blood flow images shown in FIGS. 6 and 7 are still images, alternatively, the blood flow images to be displayed on the display 2 may be moving images.

As can be seen by comparing FIGS. 6 and 7, the blood flow image 320 displayed by the ultrasound diagnostic device 100 according to the present embodiment shown in FIG. 6 emphasizes a portion of a blood flow signal and allows a blood vessel to be recognized as a tube. In addition, since adjacent blood vessels can be separated, a state of blood flow can be comprehended more accurately.

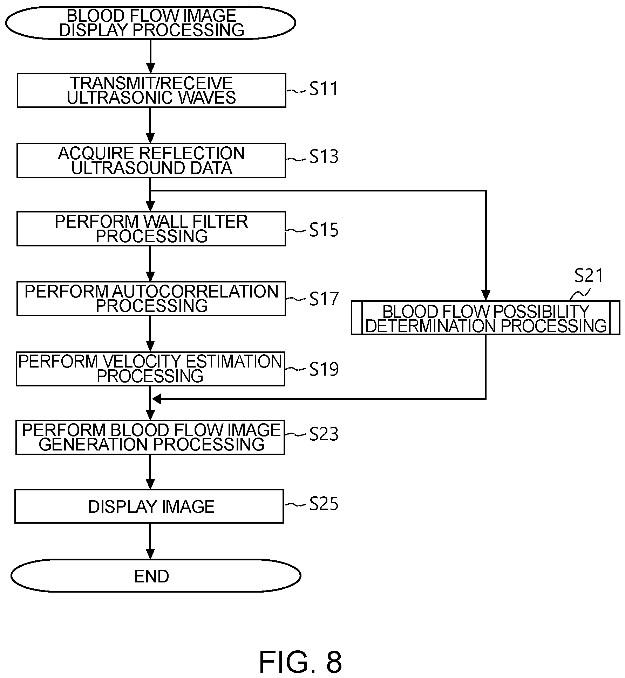

Next, blood flow image display processing executed by the ultrasound diagnostic device 100 will be described based on FIG. 8. FIG. 8 is a diagram showing a flowchart for describing blood flow image display processing executed by the Doppler processing circuit 104, the image generation circuit 107, and the control circuit 111 in the ultrasound diagnostic device 100. The blood flow image display processing shown in FIG. 8 is processing that is realized by, for example, the Doppler processing circuit 104, the image generation circuit 107, and the control circuit 111 by reading and collaboratively executing a blood flow image display processing program stored in the storage circuit 110.

In the blood flow image display processing, first, the control circuit 111 in the ultrasound diagnostic device 100 controls the transmission/reception circuit 101 to transmit ultrasonic waves from the ultrasound probe 1 to the subject P, receive reflected waves from the mobile object that is the subject P, and store the reflected waves as reflection ultrasound data in the buffer memory 102 (step S11). The reflection ultrasound data is also the ensemble data 300 described above.

Next, the power WF function 131 and the velocity/direction WF function 141 of the Doppler processing circuit 104 in the ultrasound diagnostic device 100 acquires the reflection ultrasound data stored in the buffer memory 102 (step S13). In other words, the Doppler processing circuit 104 acquires the reflection ultrasound data from the buffer memory 102.

Next, the velocity/direction WF function 141 of the Doppler processing circuit 104 in the ultrasound diagnostic device 100 extracts phase-change information by applying a wall filter for velocity/direction to the ensemble data 300 that is the reflection ultrasound data (step S15). As described above, at this point, the velocity/direction WF function 141 uses a fixed-length wall filter in a Doppler frequency space.

Next, the autocorrelation processing function 142 of the Doppler processing circuit 104 in the ultrasound diagnostic device 100 performs autocorrelation processing with respect to data extracted from the ensemble data 300 by the velocity/direction WF function 141 (step S17).

Next, the velocity/direction estimation function 143 of the Doppler processing circuit 104 in the ultrasound diagnostic device 100 estimates velocity information and direction information of a blood flow based on a result of the autocorrelation processing by the autocorrelation processing function 142 (step S19).

On the other hand, after acquiring the ensemble data 300 that is the reflection ultrasound data in step S13, the ultrasound diagnostic device 100 executes blood flow possibility determination processing (step S21). Due to the blood flow possibility determination processing, a probability of the mobile object that is included in the reflection ultrasound data being a blood flow is calculated based on the reflection ultrasound data.

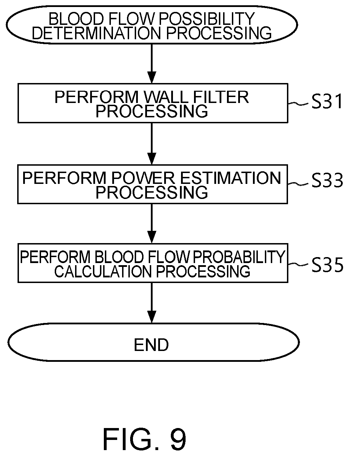

FIG. 9 is a diagram showing a flowchart for describing, in detail, contents of the blood flow possibility determination processing in step S21. The blood flow possibility determination processing is processing that is realized by, for example, the Doppler processing circuit 104 by reading and executing a blood flow possibility determination processing program stored in the storage circuit 110.

As shown in FIG. 9, in the blood flow possibility determination processing, first, the power WF function 131 of the Doppler processing circuit 104 in the ultrasound diagnostic device 100 extracts intensity information related to the mobile object by applying a wall filter for power (intensity) information to the ensemble data 300 that is the reflection ultrasound data (step S31). As described above, at this point, the power WF function 131 uses an eigenvalue expansion-type wall filter.

Next, the power estimation function 132 of the Doppler processing circuit 104 in the ultrasound diagnostic device 100 estimates intensity information of the mobile object from a component extracted from the ensemble data 300 by the power WF function 131 (step S33).

The blood flow possibility determination function 133 determines a probability that the mobile object is a blood flow based on the intensity information of the mobile object estimated by the power estimation function 132 (step S35). In the present embodiment, for example, the probability that the mobile object is a blood flow is calculated as probability information. For example, the probability information is calculated as a numerical value between 0% and 100%. By executing step S35, the blood flow possibility determination processing is completed.

Returning to FIG. 8, in the blood flow image display processing shown in FIG. 8, the image generation circuit 107 in the ultrasound diagnostic device 100 generates the blood flow image 320 based on the velocity information and the direction information of the blood flow generated in step S19 and the probability information generated in step S35 (step S23). As described above, the image generation circuit 107 generates, using the color map 400, the blood flow image 320 in which each corresponding region is colored based on the probability information that indicates the probability of being a blood flow, the velocity information, and the direction information.

Next, the image generation circuit 107 in the ultrasound diagnostic device 100 displays the generated blood flow image 320 on, for example, the display 2 (step S25). For example, in the present embodiment, the generated blood flow image 320 is stored in the image memory 109 and the blood flow image 320 is displayed on the display 2. Accordingly, the blood flow image display processing according to the present embodiment is completed.

As described above, with the ultrasound diagnostic device 100 according to the present embodiment, even a blood flow with low velocity can be appropriately displayed in the blood flow image 320 while suppressing display of clutter. In addition, a color scheme in the blood flow image 320 can be appropriately displayed based on a velocity of the blood flow. In other words, in the blood flow image 320, a blood flow with low velocity can be displayed while preventing clutter from being displayed and a validity of the velocity of a displayed blood flow can be guaranteed while separating the blood flow with low velocity from the clutter.

The embodiment described above can be modified in various ways. For example, when determining the probability of being a blood flow, dispersion information of a mobile object may be additionally used with the velocity information and the direction information of the mobile object. For example, since a small dispersion indicates that a phase change is stable, the blood flow is likely to be that of a non-pulsating blood vessel. Such information can also be used when determining the probability of being a blood flow.

According to at least one of the embodiments described above, a blood flow image that also displays a blood flow with low velocity can be generated while suppressing clutter. In addition, validity of the velocity of the blood flow displayed in the blood flow image can be guaranteed.

The term "processing circuitry" used in the description given above refers to, for example, a circuit such as a CPU (Central Processing Unit), a GPU (Graphics Processing Unit), an application-specific integrated circuit (ASIC), or programmable logic devices (for example, an simple programmable logic device (SPLD), a complex programmable logic device (CLPD), and a field programmable gate array (FPGA)). The processing circuitry realizes functions by reading and executing programs stored in a storage circuit. Note that a configuration may be adopted in which a program is directly incorporated into the processing circuitry instead of being stored in the storage circuit. In this case, the processing circuitry realizes functions by reading and executing the program incorporated into the processing circuitry. The processing circuitry is not limited to a single circuit configuration of the processor and a plurality of independent circuits may be combined to constitute a single processor and the functions may be realized by the single processor. Furthermore, a plurality of constituent elements may be integrated into a single processor of the processing circuitry and the functions may be realized by the single processor of the processing circuitry.

While certain embodiments have been described above, the embodiments have been presented by way of example only and are not intended to limit the scope of the invention. The novel device and method described in the present specification can be implemented in various other modes. In addition, various omissions, substitutions, and modifications can be made to the modes of the device and method described in the present specification to the extent that they do not depart from the gist of the invention. The appended claims and their equivalents are intended to include such modes and modifications that are included in the scope and gist of the invention.

## Claims

1. An ultrasound diagnostic device, comprising processing circuitry configured to
acquire reflection ultrasound data from a mobile object;
extract velocity information of the mobile object by applying a first filter to the reflection ultrasound data;
determine a probability that the mobile object is a blood flow based on the reflection ultrasound data and output probability information; and
generate a blood flow image based on the extracted velocity information of the mobile object and the outputted probability information.

2. The ultrasound diagnostic device according to claim 1, wherein
the processing circuitry is further configured to
extract intensity information of the mobile object by applying a second filter to the reflection ultrasound data; and
determine a probability that the mobile object is the blood flow based on the intensity information.

3. The ultrasound diagnostic device according to claim 2, wherein
the processing circuitry is further configured to determine that the higher the intensity indicated by the intensity information is, the higher the probability of being the blood flow is.

4. The ultrasound diagnostic device according to any one of claims 1 to 3, wherein
the processing circuitry is further configured to generate the blood flow image so that the higher the probability of being the blood flow is, the higher the brightness is, and the lower the probability of being the blood flow is, the lower the brightness is.

5. The ultrasound diagnostic device according to claim 4, wherein
the processing circuitry is further configured not to express a difference in velocity in the velocity information of the mobile object in terms of a difference in brightness.

6. The ultrasound diagnostic device according to claim 5, wherein
the processing circuitry is further configured to generate the blood flow image using a color map in which a first axis indicates the probability of being the blood flow and a second axis indicates the velocity of the mobile object.

7. The ultrasound diagnostic device according to claim 6, wherein
the processing circuitry is further configured to extract direction information in addition to the velocity information of the mobile object by applying the first filter to the reflection ultrasound data.

8. The ultrasound diagnostic device according to claim 7, wherein
in the color map, a first color is assigned to the mobile object moving in a direction approaching an ultrasound probe, a second color is assigned to the mobile object moving in a direction receding from the ultrasound probe, a third color is assigned to the mobile object of which a velocity of movement is zero, and a color scheme is adopted such that the first color, the second color, and the third color gradually change based on a probability of being a blood flow and the velocity of the mobile object.

9. The ultrasound diagnostic device according to claim 7, wherein
in the color map, the first color is assigned to the mobile object of which a velocity of movement is zero, the second color is respectively assigned to maximum values on the color map related to the velocity of the mobile object moving in a direction approaching an ultrasound probe and the velocity of the mobile object moving in a direction receding from the ultrasound probe, and a color scheme is adopted such that a color changes to the second color via the third color as the velocity of the mobile object increases in the direction approaching the ultrasound probe and a color changes to the second color via a fourth color as the velocity of the mobile object increases in the direction receding from the ultrasound probe.

10. The ultrasound diagnostic device according to any one of claims 2 to 9, wherein
the first filter is a fixed-length wall filter in a Doppler frequency space, and
the second filter is an eigenvalue expansion-type wall filter.
